# EUROPEAN PATENT APPLICATION

(11) **EP 3 960 153 A1**
(43) Date of publication of application: **02.03.2022**
(21) Application number: 19901402.8
(22) Date of filing: 27.11.2019
(51) Int. Cl.: A61K 8/9789, A61K 8/37, A61K 8/34, A61K 8/86, A61Q 19/08

(54) **SKINCARE COMPOSITION FOR TIGHTENING SKIN, AND PREPARATION METHOD THEREFOR**

(30) Priority: 11.07.2019 CN 201910622830
(71) Applicant: Zhejiang Kangmanjia Daily Necessities Co., Ltd., Lanxi City Jinhua City Zhejiang 321100 (CN)
(72) Inventor: ZHANG, XunTing, Jinhua City, Zhejiang 321100 (CN); ZHONG, WeiFang, Jinhua City, Zhejiang 321100 (CN)
(74) Representative: Snipe, Benjamin Thomas Fletcher
(86) International application number: PCT/CN2019/121342
(87) International publication number: WO 2021/003963

(57) **Abstract**

A skin-care composition for tightening skin and a method for preparing same. The skin-care composition is prepared from an emulsifier and an acmella oleracea extract; the emulsifier comprises stearate and stearyl alcohol emulsifier. The skin-care composition for tightening skin of the invention has good effects in tightening skin, preventing wrinkles and resisting aging, has high heat stability, cold stability and mechanical stability, can be applied to various skin-care products without causing skin irritations, and is suitable for oily and sensitive skin.

## Description

### TECHNICAL FIELD

The invention relates to the field of skin-care products, in particular to a skin-care composition for tightening skin and a method for preparing the same.

### BACKGROUND

The problem of skin aging becomes more severe with age, the aggravation of environmental pollution and the increase of daily life pressure, and people are paying more and more attention to anti-aging skin-care products. Skin aging appears as declining skin elasticity, larger pores, more skin wrinkles, coarser skin, and the like. Clearly, it is not enough to prevent against skin aging only by using moisturizer skin-care products, and products for tightening skin and enhancing skin elasticity are also needed. However, the performance of existing skin-care products for tightening skin and improving skin elasticity is yet to be improved, and the stability of such skin-care products also needs to be improved. The invention provides a skin-care composition for tightening skin. The skin-care composition for tightening skin can be applied to various skin-care products to tighten skin, prevent wrinkles, and resist skin aging, has high stability, and is suitable for people with different skin types.

### SUMMARY

To solve the above-mentioned problems, the invention provides, in the first aspect, a skin-care composition for tightening skin. The skin-care composition is prepared from an emulsifier and an acmella oleracea extract, wherein the emulsifier comprises one or more selected from stearate, stearyl alcohol, and stearyl glucoside.

In a preferred technical solution of the invention, the emulsifier further comprises stearyl glutamate that accounts for 2-4wt% of the emulsifier.

In a preferred technical solution of the invention, the stearyl glutamate accounts for 3-10wt% of the acmella oleracea extract.

In a preferred technical solution of the invention, the skin-care composition is further prepared from hydrogenated lecithin, trehalose and a hydrolyzed soy protein complex.

In a preferred technical solution of the invention, the weight ratio of the trehalose and the hydrolyzed soy protein complex is 1: (2-3).

In a preferred technical solution of the invention, the skin-care product is further prepared from a complex plant extract, a caffeine liposome and a trifluoroacetyl tripeptide-2 complex.

In a preferred technical solution of the invention, the weight ratio of the complex plant extract, the caffeine liposome and the trifluoroacetyl tripeptide-2 complex is 1:(0.5-1.5):(1.5-2.5).

In the second aspect, the invention provides an application of the skin-care composition for tightening skin to a skin-care product, wherein the skin-care product is one or more selected from a lotion, an emulsion, a face cream, an eye cream, a face cleanser, a face mask, a BB cream, a pure essence, a sunscreen scream, a sunscreen lotion, an acne lotion, a pimple lotion, a cleansing toner, a cleansing lotion, an essential oil, a shampoo, a moisturizer, a toner, an astringent and a softening lotion.

In a preferred technical solution of the invention, the skin-care product is a face cream, wherein the face cream is prepared from, by weight, 9%-25% of the skin-care composition, 0.1%-0.5% of a thickener, 15%-20% of a moisturizer, 0-0.5% of an antioxidant, 8%-20% of an emollient, 0.5%-2% of a chelator, 0.2%-0.8% of a skin conditioner, and the balance deionized water.

In the third aspect, the invention provides a method for preparing a face cream, wherein the face cream is prepared from, by weight, 9%-25% of the skin-care composition, 0.1%-0.5% of a thickener, 15%-20% of a moisturizer, 0-0.5% of an antioxidant, 8%-20% of an emollient, 0.5%-2% of a chelator, 0.2%-0.8% of a skin conditioner, and the balance deionized water;

The method for preparing the face cream comprises the following steps:

Pre-treatment of an aqueous phase: after the thickener and the deionized water are homogenized, the moisturizer, the antioxidant and half of the skin conditioner by weight are added and are then stirred and heated;

Pre-treatment of an oil phase: all components, except stearyl glutamate, of the emulsifier, and the emollient are stirred and heated, and then an acmella oleracea extract is added; and

After-treatment: the aqueous phase and the oil phase are mixed, homogenized and cooled, the chelator, the rest of the skin conditioner, and all components, except trehalose, hydrogenated lecithin and an emulsifier, of the skin-care composition are added, stirred and filtered to obtain the face cream.

Compared with the prior art, the invention has the following beneficial effects: the skin-care composition for tightening skin has good effects in tightening skin, preventing wrinkles, and resisting aging, has high heat stability, cold stability and mechanical stability, can be applied to various skin-care products without causing cause skin irritations, and is also suitable for oily and sensitive skin.

### DETAILED DESCRIPTION OF THE EMBODIMENTS

The contents of the invention can be understood more easily with reference to the following detailed description of preferred implementations and embodiments of the invention. Unless otherwise specified, all techniques and scientific terms used in this article have the meanings commonly appreciated by those ordinarily skilled in the art. In the case of a contradiction, definitions in this specification shall prevail.

For example, the term "prepare from" in this article is equivalent to "include". The term "include", "comprise", "have", "contain" or other transformations are intended to refer to non-exclusion inclusion. For example, a composition, step, method, product or device comprising elements listed is not limited to including these elements listed, and may also comprise other elements not specifically listed, or inherent elements of the composition, step, method, product or device.

The conjunction "consist of' is exclusive of any elements, steps or components that are not listed. In the case where this conjunction is applied to a claim, the claim limited by this conjunction is exclusive and does not include any material except for the materials listed, while conventional impurities relating to the materials listed are exceptions. In the case where the conjunction "consist of' is applied to a clause of the subject of a claim instead of directly following behind the subject, this conjunction only prescribes a limit to elements described in the clause, and other elements are not excluded out of the whole claim.

In the case where quantity, concentration, or other values or parameters are represented by ranges, preferred ranges, or a series of preferred upper limits and preferred lower limits, all ranges defined by any pair of upper range limits or preferred values and lower range limits or preferred values should be appreciated as specifically disclosed no matter whether or not these ranges are independently disclosed. For instance, in the case where the range from 1 to 5 is disclosed, this range intends to comprise a range from 1 to 4, a range from 1 to 3, a range from 1 to 2, ranges from 1 to 2 and from 4 to 5, and ranges from 1 to 3 and from 1 to 5. Unless otherwise specified, any value ranges involved in this article intend to comprise end values as well as all integers and factions within these ranges.

Unless otherwise clearly pointed out, an object discussed in a singular form includes a plural condition. "Optional" or "anyone" indicates that a matter or event described may occur or may not occur, and this description includes the condition where the event occurs and the condition where the event does not occur.

Approximate expressions used in the description and claims to modify quantity does not mean that the invention is limited to a specific quantity and also includes acceptable amendments close to the quantity without causing changes to related basic functions. Correspondingly, the term "approximate" or "about" used for modifying a value intends to express that the invention is not limit to an accurate value. In certain embodiments, the approximate expressions may correspond to the precision of instruments used for value measurement. In the description and claims of this application, range limits can be combined and/or interchanged, and unless otherwise stated, these ranges include all sub-ranges thereof.

In addition, indefinite articles such as "one" in front of elements or components of the invention prescribes no limit to the quantity (the frequency of occurrence) of the elements or components. Therefore, "one" should be interpreted as "one" or "at least one", and unless in the case where a quantity obviously refers to a singular form, any element or component defined by the singular form also includes the plural form.

The invention is explained below with reference to specific implementations, but the invention is not limited to the specific embodiments given below.

In the first aspect, the invention provides a skin-care composition for tightening skin. The skin-care composition is prepared from an emulsifier and an acmella oleracea extract, wherein the emulsifier comprises stearate and stearyl alcohol.

### Emulsifier

The emulsifier of the invention comprises one or more selected from stearate, stearyl alcohol, and stearyl glucoside.

As an example, the stearate includes, but is not limited to, glycol stearate, glyceryl stearate, and polyoxyethylene stearate.

As an example, the stearyl alcohol includes, but is not limited to, cetostearyl alcohol, cetostearyl alcohol polyether and cetostearyl alcohol ester.

As an example, the stearyl glucoside includes, but is not limited to, stearyl glucoside and isostearyl glucoside.

Preferably, the emulsifier comprises stearate emulsifier and/or stearyl alcohol emulsifier.

More preferably, the emulsifier comprises glyceryl stearate/PEG-100, stearate and cetostearyl alcohol.

Due to great differences in structure, solubility and diffusivity of all components in a composition and a skin-care product, the components cannot be dispersed easily when the skin-care product such as a paste, a cream and a lotion is prepared, and the skin-care product is prone to agglomeration and caking under a specific condition such as a high temperature or a low temperature. The applicant finds that the stability of the composition and the skin-care product can be regulated and controlled by setting parameters, such as the components and proportion, of an emulsifier in the composition, so as to avoid the instabilities such as caking and thinning of the composition and the skin-care product under a high-temperature or a low-temperature. The applicant finds that stable skin-care products can be easily obtained if stearate, stearyl alcohol and stearyl glucoside, particularly glyceryl stearate, stearate PEG-100 and cetostearyl alcohol, is used as the emulsifier, which is possibly because stable micelles of a proper size and a stable structure are formed under the interaction of hydrophilic ends and hydrophobic ends of these components and other components in the composition and the skin-care product are dispersed by the micelles to form stable emulsion particles, and thus, the composition has good stability.

Preferably, the emulsifier further comprises stearyl glutamate that accounts for 2-4wt% of the emulsifier.

More preferably, the stearyl glutamate of the invention is sodium stearoyl glutamate.

The applicant finds that the emulsifier containing a certain quantity of stearyl glutamate can further improve the stability of the composition, so that the skin-care product containing the composition can have a stable structure and is not prone to agglomeration or caking under a high or low temperature or a strong centrifugal force. Because of the nonionic structures of stearate and stearyl alcohol, although micelles can be formed by means of the hydrophilic-ydrophobic structures to disperse other components, the adjacent micelles have high affinity and adsorptivity, and consequentially, emulsion particles of the micelles are likely to agglomeration and caking after the system acquires sufficient activation energy from the outside. While the wall thickness of the micelles can be improved after a certain quantity of stearyl glutamate is added, so that the sensitivity of the micelles to the external environment is reduced; and meanwhile, surface charge distribution of the micelles can be changed by adding stearyl glutamate, and thus, the agglomeration between the adjacent micelles is avoided under the repulsive effect of identical charges.

The applicant also finds that the dosage of stearyl glutamate should be strictly controlled, and the stability of the composition and the skin-care product will be reduced if too much or too little stearyl glutamate is added, which is possibly because stearyl glutamate can change the structure of the micelles and the structure of the micelles may be destroyed by an inappropriate dosage of stearyl glutamate and may become loose, and consequentially, instability is caused. Meanwhile, the applicant finds that the same effect cannot be achieved if the stearyl glutamate is replaced with other conventional anionic surfactants, which is possibly because the stabilizing effect of the stearyl glutamate on the composition is also related to the carbon chain structure of the stearyl glutamate.

### Acmella oleracea extract

The acmella oleracea extract of the invention is rich in isobutyramide and extremely-active spilanthol and can interact with fibroblasts of the corium layer and a collagen network to activate the natural activity and biomechanic function of the fibroblasts to reform the structure of the corium layer, so that the density and firmness of skin are rapidly improved, the coarseness of the skin is reduced, the smoothness of skin is promoted, crow's feet at the eye corners are reduced, and a remarkable anti-wrinkle effect is achieved. The acmella oleracea extract also has an anti-bacterial effect and a skin whitening effect. The acmella oleracea extract of the invention contains a small quantity of octanoic acid/capric triglyceride.

Preferably, the acmella oleracea extract of the invention is purchased from PUEN Biotech (Shanghai) Co., Ltd., under the designation of Gatuline IN-Tense.

The applicant finds that the composition added with a certain quantity of the acmella oleracea extract can greatly reduce expression wrinkles and improve the smoothness and firmness of skin. The acmella oleracea extract containing a certain quantity of octanoic acid/capric triglyceride can be easily dispersed in the composition and the skin-care product. The acmella oleracea extract contains a large quantity of cocamidopropyl betaine compounds which can be absorbed by skin to stimulate the biomechanic function of the fibroblasts to reform the dermal structure of the corium layer. By means of the powerful absorption between strong polar groups such as amido bonds in these cocamidopropyl betaine compounds and skin cells, the thickness of the corium layer is improved, and the structure of the corium layer is strengthened, thus avoiding cutis laxa and wrinkles and keeping skin smooth and firm.

Preferably, the stearyl glutamate accounts for 3-10wt% of the acmella oleracea extract.

The applicant accidentally finds that the composition has a poor skin smoothening and firming effect and a poor anti-wrinkle effect when not containing a certain quantity of stearyl glutamate, and cannot realize an effect until after being used for a long time. When the composition contains stearyl glutamate accounting for about 3-10wt% of the acmella oleracea extract, these effects can be greatly improved, which is possibly because the stearyl glutamate has a great influence on the permeation of the acmella oleracea extract into skin and the absorption of the acmella oleracea extract by skin cells. If the composition does not contain the stearyl glutamate, the acmella oleracea extract may stay in the epidermal layer and cannot permeate into the corium layer, or is slowly absorbed by the corium layer, and in this case, it will take a long time to stimulate sufficient cell activity to fulfill a remarkable effect.

In a preferred embodiment, the composition for the skin-product is further prepared from hydrogenated lecithin, trehalose and a hydrolyzed soy protein complex.

### Hydrogenated lecithin

The hydrogenated lecithin of the invention is a stable substance which is formed by hydrogenation of lecithin under the effect of a catalyst. Lecithin is an indispensable substance for each cell, and the lack of lecithin will reduce the regeneration capacity of skin cells and lead to skin coarseness and wrinkles. The regeneration capacity of skin can be guaranteed after the skin absorbs a proper quantity of lecithin, and the skin will become shiny by means of good hydrophily and lipophilicity of lecithin. In addition, lecithin molecules are arrayed on two layers to form a selective and protective screen which is of great importance in controlling the cell life, activating cells and maintaining metabolism, resisting anti-oxidative damage, fulfilling after-sun recovery, beautifying the skin, and resisting aging. The hydrogenated lecithin reserves all active components of lecithin, has better stability and emulsibility and good hydrophily and moisture retention, is friendly to skin and mucous membranes, and can be applied to a cosmetic formula to realize moisturizing, emulsifying, dispersing and anti-oxidization effects. In addition, the hydrogenated lecithin can also serve as a surfactant to condition skin to maintain oil-moisture balance of the skin, so that the skin can better absorb nutrients. The hydrogenated lecithin can be applied to various cosmetics.

### Trehalose

The trehalose of the invention is a safe and natural carbohydrate and can effectively protect the structure of epidermal cytomembranes, activate cells and condition skin to keep the skin health, natural and elastic. The trehalose can form a special protective membrane on cell surfaces to protect epidermal cells against moisture losses in a hot, cold, dry, or strong ultraviolet radiation environment, which may otherwise cause damage to the skin; the original nutrients and moisture of the skin are maintained, sunburns and melanin pigmentation of the skin are avoided, and skin aging is effectively resisted; and mucus separated out of the membrane can mildly moisturize the skin and radiate external heat, and thus, the skin is protected against damage and is kept bright, shiny and soft.

Preferably, the hydrogenated lecithin accounts for 15-30wt% of the trehalose.

### Hydrolyzed soy protein complex

The hydrolyzed soy protein complex of the invention includes hydrolyzed soy protein which is rich in HRGPs which can promote the formation of skin collagen to keep the skin smooth and tight; meanwhile, the hydrolyzed soy protein has an effective moisturizing effect and can induce the skin to generate hyaluronic acid; with the decline of the content of hyaluronic acid, the skin becomes mature and aged, in this case, the hydrolyzed soy protein serves as an induction element to increase the concentration of hyaluronic acid in the skin so as to fulfill an effective moisturizing effect. The hydrolyzed soy protein complex of the invention further includes acacia senegal gum and xanthan gum. The content of specific components of the hydrolyzed soy protein complex is not further limited in the invention.

Preferably, the weight ratio of the trehalose and the hydrolyzed soy protein complex is 1 : (2-3).

The applicant finds that although micro-molecular alcohols such as 1,3-propanediol and methylpropanediol, and active components such as hydroxyacetophenone can fast permeate into the cuticle of skin to react with cell tissue of the skin to moisturize and condition the skin, the violent reaction between these components and the skin may lead to a strong stress reaction of the skin when the composition is used, and particularly, because of the sensitivity of oily skin and sensitive skin to components such as micro-molecular alcohols and hydroxyacetophenone in the composition, inflammation and allergies of the skin may be caused, and side reactions such as pimples in a large area may also be caused. In view of this, the applicant adds a certain quantity of hydrolyzed soy protein complex, hydrogenated lecithin and trehalose to the skin-care composition to regulate the activity of the composition, so that the composition can keep the skin elastic and tight and can also mildly act on oily skin and sensitive skin to avoid direct contact of high-concentration high-activity chemical components in the skin-care product with the skin, which may otherwise cause a series of side reactions such as allergies and inflammation. This is possibly because due to the special molecular structures of the hydrogenated lecithin and trehalose, the hydrogenated lecithin has high hydrophily and hydrophobicity and can form a micellar structure which reacts with the polyhydroxyglucoside structure of the trehalose to form a spatial network structure, and groups such as hydroxyl groups and phosphate groups in the structure form cavity structures of different sizes by means of hydrogen bonds or other acting force to wrap the high-activity chemical compounds such as micro-molecular alcohols in the skin-care product, so that the high-concentration active components permeate into the skin with the skin-care product to be absorbed by the cell tissue of the skin without making direct contact with the skin, and are then slowly released to fulfill corresponding effects, and allergies and other side effects are avoided.

In addition, the molecular structures of the hydrolyzed soy protein, the acacia Senegal gum and the xanthan gum contained in the hydrolyzed soy protein complex have many polar groups such as hydroxyl groups and amino groups, molecules are likely to form a compact cluster structure under the effect of highly-active hydrogen bonds in the molecules and cannot permeate into skin easily, and the absorption speed is low. However, by compounding the hydrolyzed soy protein complex with the hydrogenated lecithin and the trehalose in a certain proportion, the acting force in the molecules can be weakened to a certain extent, so that the molecule chains of these components are further extended to be more friendly to skin tissue and be absorbed by skin cells more rapidly to improve the thickness and elasticity of the cuticle, and accordingly, the tightness of the skin is improved, and wrinkles are reduced.

More preferably, the hydrolyzed soy protein complex of the invention is purchased from Shanghai YaoLan Trading Co., Ltd. under the designation of Firming Toner.

In a preferred implementation, the skin-care composition of the invention is further prepared from a complex plant extract, a caffeine liposome and a trifluoroacetyl tripeptide-2 complex.

### Complex plant extract

The complex plant extract comprises an aloe vera leaf extract and a brassica oleracea italica extract.

The aloe vera leaf extract has bioactivity in various aspects, is an important additive of cosmetics, has good skin-activating and oxidation resisting effects, can promote the growth of fibrous protein and synthesis of collagen and scavenge free radicals, can promote the synthesis of cholesterol, change the compositions of sebum, reduce oil and improve the softness of the skin, and has antibacterial, anti-inflammation effects as well as a moisturizing effect.

The brassica oleracea italica extract is extracted from brassica oleracea italic and can promote wound healing. When applied to the skin-care product, the brassica oleracea italica extract has anti-oxidation, anti-wrinkle and anti-stress effects and can assist in recovery of damaged skin.

Preferably, the complex plant extract of the invention is purchased from Shanghai RuiMu Chemical Co., Ltd. under the designation of BioDtox.

The applicant finds that the complex plant extract of the invention may include various components such as an anthraquinone compound, aloe polysaccharides, bioflavonoid and alkaloid, which can permeate into skin via pores to remove dirt and grease in the pores, and can, after being absorbed by skin tissue, regulate the secretion of sebaceous glands and prevent the pore blockage caused by excessive grease secretion of the sebaceous glands or even pore coarseness caused by pore inflammation.

### Caffeine liposome

The caffeine liposome of the invention includes caffeine which contains active components such as antioxidants and can resist and restrain free radicals, thus delaying skin aging, preventing redness, protecting the skin against ultraviolet damage, and improving the skin color. The caffeine can dehydrate adipose cells, thus reducing lipid globules on the skin surface and smoothing the skin.

The caffeine liposome of the invention further includes nicotinamide, lecithin and phenoxyethanol, and the caffeine is wrapped in micelles formed by lecithin by means of the hydrophily and hydrophobicity of the lecithin, so that invalidation caused by early transformations of the structure of the caffeine is avoided; and by means of the good affinity of the lecithin to skin tissue and the interaction between the lecithin and the skin tissue, these components can permeate into the skin more rapidly to be absorbed by the skin tissue, thus eliminating grease and edema more rapidly and improving the condition of the skin.

Preferably, the caffeine liposome of the invention is purchased from Shanghai HaoYun Chemical Co., Ltd. under the designation of LIPO COFFIN HERBASOME

### Trifluoroacetyl tripeptide-2 complex

The trifluoroacetyl tripeptide-2 complex of the invention includes trifluoroacetyl tripeptide-2. The trifluoroacetyl tripeptide-2 is an active peptide consisting of three amino acids and has a sequence of N-(2,2,2-trifluoroacetyl)-Val-Tyr-Val-OH, and thanks to the special molecular structure of the trifluoroacetyl tripeptide-2 and the affinity of the trifluoroacetyl tripeptide-2 to the skin tissue environment, the trifluoroacetyl tripeptide-2 can be absorbed by skin tissue to restrain the generation of MMP-1 irritation ECM and to delay the aging of skin cells and skin wrinkles. The trifluoroacetyl tripeptide of the invention further includes a small quantity of glycerinum and glucan.

Preferably, the trifluoroacetyl tripeptide-2 complex of the invention is purchased from Shanghai ZhiRou Chemical Co., Ltd. under the designation of Progline.

More preferably, the weight ratio of the complex plant extract, the caffeine liposome and the trifluoroacetyl tripeptide-2 complex is 1 : (0.5-1.5) : (1.5-2.5).

The applicants finds that when a certain quantity of complex plant extract, caffeine liposome and trifluoroacetyl tripeptide-2 complex is added to the composition, the composition can smoothen and tighten the skin, rapidly eliminate grease and edema, and can also remarkably improve the elasticity of the skin and remove wrinkles. The performance of the composition is particularly remarkable when the weight ratio of the complex plant extract, the caffeine liposome and the trifluoroacetyl tripeptide-2 complex is 1 : (0.5-1.5) : (1.5-2.5).

This is because when the weight ratio of the complex plant extract, the caffeine liposome and the trifluoroacetyl tripeptide-2 complex is within a certain range, the optimum balance is maintained under the synergistic action of corresponding components in the composition, the complex plant extract can clean and soften the cuticle and can promote the caffeine liposome to permeate into the skin more rapidly while the pores are minimized, the activity and permeability of the caffeine liposome wrapped in the lecithin are improved, the lecithin is similar to the skin tissue in structure and thus can be reversed in the permeation process to change the skin structure, absorption of the trifluoroacetyl tripeptide-2 complex is promoted, and under the synergistic action of these components, the composition can be rapidly absorbed by skin tissue, so that the structure of the corium layer of the skin is enhanced, generation of collagen is promoted, a cross-linked spatial network is formed under the effect of corresponding enzymes in the skin, the elasticity of the skin is improved, and wrinkles are removed. The skin is kept smooth and tight.

On the second aspect, the invention provides an application of the skin-care composition for tightening skin to a skin-care product, wherein the skin-care product is one or more selected from a lotion, an emulsion, a face cream, an eye cream, a face cleanser, a face mask, a BB cream, a pure essence, a sunscreen scream, a sunscreen lotion, an acne lotion, a pimple lotion, a cleansing toner, a cleansing lotion, an essential oil, a shampoo, a moisturizer, a toner, an astringent and a softening lotion

The emulsion and the face cream in the invention are liquid cream cosmetics. The face cleanser, the cleansing toner and the cleansing lotion generally refer to liquid products consisting of oil-phase substances, aqueous-phase substances, surfactants, moisturizers, nutrients, and other components and include, but are not limited to, face cleansers, cleansing pastes and cleansing lotions. The facemask is a carrier of skin-care products and includes, but is not limited to, a paste mask, a tear-type mask, a gel mask, and a tissue mask. The BB cream refers to Blemish Balm and has, but is not limited to, the functions of covering skin spots, protecting the skin against sunburns and minimizing pores. The pure essence refers to condensed nourishing substances and includes, but is not limited to, an essence toner, an essence lotion, an essence mask, an essence injection and an essence capsule. The sunscreen cream is a cosmetic containing a sun-screening agent capable of shielding or absorbing ultraviolet to protect the skin against sunburns. The acne lotion is a liquid substance having the function of curing acne. The pimple lotion is a liquid substance having the function of curing pimples.

In a preferred implementation, the skin-care product of the invention is a face cream, wherein the face cream is prepared from, by weight, 9% - 25% of the skin-care composition, 0.1% - 0.5% of a thickener, 15% - 20% of a moisturizer, 0 - 0.5% of an antioxidant, 8% - 20% of an emollient, 0.5% - 2% of a chelator, 0.2% - 0.8% of a skin conditioner, and the balance deionized water.

The face cream provided by the invention is a white or faint yellow fine paste and has a specified fragrance (rose), a pH value from 5.50 to 6.50, and a relative density of 1.00±0.02g/cm³ under 20°C.

The pH value of the invention is obtained by a pH meter through a pH test method according to GB T13531.1-2008.

The relative density of the invention is obtained by a density gauge through a relative density test method according to GB T13531.4-2013.

### Thickener

Preferably, the thickener of the invention is one or more selected from acrylic ester, PTPPHMA copolymer or salt thereof, carboxyvinyl polymer or salt thereof, alginate, polyol ester of alginic acid, polyvinylpyrrolidone, polyvinyl alcohol, sodium acrylate, sodium tripolyphosphate, carrageenan, Carbomer, xanthan gum and hydroxy propyl cellulose.

### Moisturizer

Preferably, the moisturizer of the invention is one or more selected from polyhydric alcohols, natural polysaccharide, hydrophilic synthetic polymers, and glycine betaine.

As an example, the polyhydric alcohols of the moisturizer include, but are not limited to, glycerinum, 1,3-propanediol, 2-methyl-1,3-propanediol, 1,4-butanediol, 1,3-butanediol, 1,5-butanediol, 1,5-pentanediol, diglycerol, erythritol, gluconic acid, 1,2,6-hexanetriol, inositol, lactitol, maltitol, mannite, xylitol and trehalose.

As an example, the natural polysaccharide of the moisturizer includes, but is not limited to, hyaluronic acid, acetyl hyaluronic acid, heparin, glucuronic acid, chondroitin, chondroitin sulfate, pectin and alkali metal salts thereof in proper cases.

As an example, the hydrophilic synthetic polymers of the moisturizer include, but are not limited to, poly(2-methacryloyloxyethyl phosphorylcholine) and poly(2-methylacryloyloxy ethyl phosphoryl-CO-methacrylamide).

As an example, the glycine betaine of the moisturizer includes, but is not limited to, glycine betaine and derivates thereof, and any compounds contained in zwitterionic compounds of corresponding sulphobetaine (carboxyl of the glycine betaine is replaced with sulfo), such as decyl glycine betaine, lauryl glycine betaine, myristyl glycine betaine, cetyl glycine betaine, stearoyl glycine betaine, behenic glycine betaine, lauramide propyl glycine betaine, oleamide propyl glycine betaine, palmitamide propyl glycine betaine, lauryl sulphobetaine, coco-sulphobetaine, poly(methylacryloyloxy ethyl glycine betaine), and poly (methylacryloyloxy ethyl glycine betaine-co-2-ethoxyl-methacrylic acid).

More preferably, the moisturizer of the invention comprises methylpropanediol and 1,3-propanediol, and glycine betaine.

### Antioxidant

The antioxidant is one or more selected from an oil-soluble antioxidant, a water-soluble antioxidant, and a natural extract containing the antioxidant.

As an example, the water-soluble antioxidant includes, but is not limited to, a sulfhydryl compound and derivates thereof, lipoic acid, dihydrolipoic acid, resveratrol, acetyl-cysteine, lactoferrin, and ascorbic acid and derivates thereof.

As an example, the oil-soluble antioxidant includes, but is not limited to, butylated hydroxytoluene, retinoid, tocotrienols and ubiquinone.

As an example, the natural extract containing the antioxidant includes, but is not limited to, extracts containing flavonoid, isoflavone and derivates thereof, and extracts containing resveratrol.

### Emollient

Preferably, the emollient of the invention is one or more selected from cyclopentasiloxane, polydimethylsiloxane, caprylyl methicone, tocopheryl acetate, cyclohexasioxane, diisopropyl sebacate, dicaprylyl carbonate, triglyceride(ethylhexoic acid) ester, isododecane, isononyl isononanoate, cetearyl ethyl hexanoate, butyrospermum parkii resin, simmondsia chinensis seed oil, squalane, petrolatum, and sodium PEG-7 olive oil carboxylate.

More preferably, the emollient of the invention comprises butyrospermum parkii resin, simmondsia chinensis seed oil, squalane, polydimethylsiloxane, caprylyl methicone and tocopheryl acetate.

### Chelator

Preferably, the chelator of the invention is one or more selected from ethylene diamine tetraacetic acid sodium salt, citric acid and salt thereof, phosphate, 1-diphosphonic acid and salt thereof, and caprylhydroxamic acid.

More preferably, the chelator of the invention is caprylhydroxamic acid.

The caprylhydroxamic acid of the invention contains 1,3-propanediol and 1,2-hexanediol. As a multifunctional additive, the caprylhydroxamic acid is added to the skin-care product. In addition to serving as the chelator, the caprylhydroxamic acid also has the moisturizing, skin moistening, anti-allergy, anti-oxidant and antisepsis effects. In a preferred implementation, the chelator is purchased from Shanghai HaoYun Chemical Co., Ltd. under the designation of PHL.

### Skin conditioner

The skin conditioner of the invention is not further limited in the invention. In a preferred implementation, the skin conditioner comprises allantoin, arginine and rose oil.

The allantoin is an amphoteric compound, can be combined with various substances to form a double salt, has the effects of light shielding, sterilization, antisepsis, pain relieving and oxidation prevention as well as the effects of protecting tissue, hydrophily, water absorption and preventing water dispersion, can retain the moisture in the skin, moisten the skin and maintain the skin soft, and is widely applied to freckle creams, pimple lotions, shampoos, soap, toothpaste, shaving lotions, hair conditioners, astringents, and anti-sweat and deodorant agents. The allantoin can promote the growth of tissue and metabolism of cells, and soften protein of the cuticle.

The arginine is an alkaline amino acid and can cooperate with tartaric acid and trehalose with a moisturizing function to retain the moisture of the skin and soften the skin. The arginine is the most common cosmetic nutrient auxiliary.

The rose oil is a high-grade condensed essence, can improve the fragrance of the product and eliminate objectionable odor, has the function of removing free radicals, can be used as an antioxidant to resist aging, can enhance the activity of cells and prevent wrinkles, can restrain tyrosinase to a certain extent, and has an auxiliary skin whitening effect.

In the third aspect, the invention provides a method for preparing the face cream. The face cream is prepared from, by weight, 9% - 25% of the skin-care composition, 0.1% - 0.5% of a thickener, 15% - 20% of a moisturizer, 0 - 0.5% of an antioxidant, 8% - 20% of an emollient, 0.5% - 2% of a chelator, 0.2% - 0.8% of a skin conditioner, and the balance deionized water.

The method for preparing the face cream comprises the following steps:

Pre-treatment of an aqueous phase: after the thickener and the deionized water are homogenized, the moisturizer, the antioxidant and half of the skin conditioner by weight are added and are then stirred and heated;

Pre-treatment of an oil phase: all components, except stearyl glutamate, of the emulsifier, and the emollient are stirred and heated, and then the acmella oleracea extract is added; and

After-treatment: the aqueous phase and the oil phase are mixed, homogenized and cooled, the chelator, the rest of the skin conditioner and all components, except the trehalose, the hydrogenated lecithin and the emulsifier, of the skin-care composition are added, stirred and filtered to obtain the face cream.

In a preferred implementation of the invention, in the step of pre-treatment of the aqueous phase, the homogenizing frequency is 15 - 25Hz.

In a preferred implementation of the invention, in the step of pre-treatment of the aqueous phase, the stirring frequency is 25 - 35Hz after the moisturizer, the antioxidant and half of the skin conditioner by weight are added.

In a preferred implementation of the invention, in the step of pre-treatment of the aqueous phase, heating is carried out to 80 - 85°C.

In a preferred implementation of the invention, in the step of pre-treatment of the aqueous phase, after heating to 80 - 85°C, the stearyl glutamate is added.

In a preferred implementation of the invention, in the step of pre-treatment of the oil phase, the stirring frequency is 20 - 30Hz.

In a preferred implementation of the invention, in the step of pre-treatment of the oil phase, heating is carried out to 80 - 85°C.

In a preferred implementation of the invention, in the step of after-treatment, the homogenizing frequency is 20 - 30Hz, and the homogenizing time is 8-15min.

In a preferred implementation of the invention, in the step of after-treatment, the aqueous phase and the oil phase are cooled to 40-50°C after being homogenized, and the chelator, the rest of the skin conditioner and all components, except the trehalose, the hydrogenated lecithin and the emulsifier, of the skin-care composition are added and are stirred.

In a preferred embodiment, in the step of after-treatment, the stirring frequency is 25-35Hz.

In a preferred embodiment, in the step of after-treatment, a filter cloth with 200-300 meshes is used for filtration.

The number of meshes in the invention refers to the number of meshes in one inch of the filter cloth.

### Embodiments

The invention is specifically described below in combination with embodiments. It should be noted that the following embodiments are only used to further explain the invention and should not be interpreted as limitations of the protection scope of the invention. Some non-essential improvements and adjustments made by those skilled in the art according to the contents of the invention should also fall within the protection scope of the invention.

### Example 1

Example 1 of the invention provides a skin-care composition for tightening skin. The skin-care composition for tightening skin comprises, by weight, 6.5 parts of an emulsifier and 3 parts of an acmella oleracea extract.

The emulsifier consists of, by weight, 2 parts of glyceryl stearate/PEG-100 stearate and 4.5 parts of cetostearyl alcohol (CAS No.67762-27-0).

The glyceryl stearate/PEG-100 stearate is purchased from Shanghai Goodway industrial Co., Ltd. under the designation of ARLACEL 170.

The acmella oleracea extract is purchased from PUEN Biotech (Shanghai) Co., Ltd., under the designation of Gatuline IN-Tense.

This example provides a face cream including the skin-care composition for tightening skin. The face cream is prepared from, by weight, 9.5% of the skin-care composition, 0.4% of a thickener, 16% of a moisturizer, 0.2% of an antioxidant, 11.1% of an emollient, 1% of a chelator, 0.48% of a skin conditioner, and the balance deionized water.

The thickener is Carbomer (CAS No.9007-20-9).

The moisturizer consists of, by weight, 8 parts of methylpropanediol (CAS No.2163-42-0) and 8 parts of 1,3-propanediol (CAS No.504-63-2).

The antioxidant is p-hydroxyacetophenone (CAS No.99-93-4).

The emollient consists of, by weight, 2 parts of butyrospermum parkii resin, 1 part of simmondsia chinensis seed oil, 1 part of squalane (CAS No.111-01-3), 5 parts of polydimethylsiloxane (CAS No. 9006-65-9), 2 parts of caprylyl methicone (CAS No. 17955-88-3), and 0.1 parts of tocopheryl acetate.

The butyrospermum parkii resin is purchased from Sanrise (Shanghai) Chemical Co., Ltd.

The simmondsia chinensis seed oil is purchased from FloraSun (Shanghai) Corporation.

The tocopheryl acetate is purchased from Shanghai ShangDe Chemical Co., Ltd.

The chelator is purchased from Shanghai HaoYun Chemical Co., Ltd. under the designation of PHL.

The skin conditioner consists of, by weight, 0.2 parts of allantoin (CAS. No.97-59-6), 0.2 parts of arginine (CAS No.74-79-3), and 0.08 parts of rose oil.

The rose oil is purchased from Ungerer And Company (Shanghai) Co., Ltd.

This example further provides a method for preparing the face cream. The method comprises the following steps:

Pre-treatment of an aqueous-phase: the thickener is added into the deionized water to be homogenized under 20Hz, and then the moisturizer, the allantoin and the antioxidant are added, stirred under 30Hz and are heated to 83°C;

Pre-treatment of an oil phase: the emulsifier and the emollient are stirred under 25Hz and are heated to 83°C, and then the acmella oleracea extract is added; and

After-treatment: the aqueous phase and the oil phase are mixed and homogenized for 10min under 25Hz and are then cooled to 45°C, the chelator, the arginine and the rose oil are added and are stirred under 30Hz, and after it is detected by sampling as qualified, filtration is carried out by a 200-mesh filter cloth to obtain the face cream.

### Example 2

Example 2 of the invention provides a skin-care composition for tightening skin. The skin-care composition for tightening skin comprises, by weight, 6.7 parts of an emulsifier and 3 parts of an acmella oleracea extract.

The emulsifier consists of, by weight, 2 parts of glyceryl stearate/PEG-100 stearate, 4.5 parts of cetostearyl alcohol (CAS No.67762-27-0), and 0.2 parts of sodium stearoyl glutamate (CAS No.38517-23-6).

The glyceryl stearate/PEG-100 stearate is purchased from Shanghai Goodway industrial Co., Ltd. under the designation of ARLACEL 170.

The acmella oleracea extract is purchased from PUEN Biotech (Shanghai) Co., Ltd., under the designation of Gatuline IN-Tense.

This example provides a face cream including the skin-care composition for tightening skin. The face cream is prepared from, by weight, 9.7% of the skin-care composition, 0.4% of a thickener, 16% of a moisturizer, 0.2% of an antioxidant, 11.1% of an emollient, 1% of a chelator, 0.48% of a skin conditioner, and the balance deionized water.

The thickener is Carbomer (CAS No.9007-20-9).

The moisturizer consists of, by weight, 8 parts of methylpropanediol (CAS No.2163-42-0) and 8 parts of propanediol (CAS No.504-63-2).

The antioxidant is p-hydroxyacetophenone (CAS No.99-93-4).

The emollient consists of, by weight, 2 parts of butyrospermum parkii resin, 1 part of simmondsia chinensis seed oil, 1 part of squalane (CAS No.111-01-3), 5 parts of polydimethylsiloxane (CAS No. 9006-65-9), 2 parts of caprylyl methicone (CAS No. 17955-88-3), and 0.1 parts of tocopheryl acetate.

The butyrospermum parkii resin is purchased from Sanrise (Shanghai) Chemical Co., Ltd.

The simmondsia chinensis seed oil is purchased from FloraSun (Shanghai) Corporation.

The tocopheryl acetate is purchased from Shanghai ShangDe Chemical Co., Ltd.

The chelator is purchased from Shanghai HaoYun Chemical Co., Ltd. under the designation of PHL.

The skin conditioner consists of, by weight, 0.2 parts of allantoin (CAS. No.97-59-6), 0.2 parts of arginine (CAS No.74-79-3), and 0.08 parts of rose oil.

The rose oil is purchased from Ungerer And Company (Shanghai) Co., Ltd.

This example further provides a method for preparing the face cream. The method comprises the following steps:

Pre-treatment of an aqueous-phase: the thickener is added into the deionized water to be homogenized under 20Hz, then the moisturizer, the allantoin and the antioxidant are added, stirred under 30Hz and are heated to 83°C, and then the sodium stearoyl glutamate is added;

Pre-treatment of an oil phase: all components, except the sodium stearoyl glutamate, of the emulsifier and the emollient are stirred under 25Hz and are heated to 83°C, and then the acmella oleracea extract is added; and

After-treatment: the aqueous phase and the oil phase are mixed and homogenized for 10min under 25Hz and are then cooled to 45°C, the chelator, the arginine and the rose oil are added and are stirred under 30Hz, and after it is detected by sampling as qualified, filtration is carried out by a 200-mesh filter cloth to obtain the face cream.

### Example 3

Example 3 of the invention provides a skin-care composition for tightening skin. The skin-care composition for tightening skin comprises, by weight, 6.7 parts of an emulsifier, 3 parts of an acmella oleracea extract, 0.2 parts of hydrogenated lecithin (CAS No.92128-87-5), 1 part of trehalose (CAS No.99-20-7), and 2.5 parts of a hydrolyzed soy protein complex.

The emulsifier consists of, by weight, 2 parts of glyceryl stearate/PEG-100 stearate, 4.5 parts of cetostearyl alcohol (CAS No.67762-27-0), and 0.2 parts of sodium stearoyl glutamate (CAS No.38517-23-6).

The glyceryl stearate/PEG-100 stearate is purchased from Shanghai Goodway industrial Co., Ltd. under the designation of ARLACEL 170.

The acmella oleracea extract is purchased from PUEN Biotech (Shanghai) Co., Ltd., under the designation of Gatuline IN-Tense.

The hydrolyzed soy protein complex is purchased from Shanghai YaoLan Trading Co., Ltd. under the designation of Firming Toner.

This example provides a face cream including the skin-care composition for tightening skin. The face cream is prepared from, by weight, 13.4% of the skin-care composition, 0.4% of a thickener, 16% of a moisturizer, 0.2% of an antioxidant, 11.1% of an emollient, 1% of a chelator, 0.48% of a skin conditioner, and the balance deionized water.

The thickener is Carbomer (CAS No.9007-20-9).

The moisturizer consists of, by weight, 8 parts of methylpropanediol (CAS No.2163-42-0) and 8 parts of propanediol (CAS No.504-63-2).

The antioxidant is p-hydroxyacetophenone (CAS No.99-93-4).

The emollient consists of, by weight, 2 parts of butyrospermum parkii resin, 1 part of simmondsia chinensis seed oil, 1 part of squalane (CAS No.111-01-3), 5 parts of polydimethylsiloxane (CAS No. 9006-65-9), 2 parts of caprylyl methicone (CAS No. 17955-88-3), and 0.1 parts of tocopheryl acetate.

The butyrospermum parkii resin is purchased from Sanrise (Shanghai) Chemical Co., Ltd.

The simmondsia chinensis seed oil is purchased from FloraSun (Shanghai) Corporation.

The tocopheryl acetate is purchased from Shanghai ShangDe Chemical Co., Ltd.

The chelator is purchased from Shanghai HaoYun Chemical Co., Ltd. under the designation of PHL.

The skin conditioner consists of, by weight, 0.2 parts of allantoin (CAS. No.97-59-6), 0.2 parts of arginine (CAS No.74-79-3), and 0.08 parts of rose oil.

The rose oil is purchased from Ungerer And Company (Shanghai) Co., Ltd.

This example further provides a method for preparing the face cream. The method comprises the following steps:

Pre-treatment of an aqueous-phase: the thickener is added into the deionized water to be homogenized under 20Hz, then the moisturizer, the allantoin, the trehalose, the hydrogenated lecithin and the antioxidant are added, stirred under 30Hz and are heated to 83°C, and then the sodium stearoyl glutamate is added;

Pre-treatment of an oil phase: all components, except the sodium stearoyl glutamate, of the emulsifier and the emollient are stirred under 25Hz and are heated to 83°C, and then the acmella oleracea extract is added; and

After-treatment: the aqueous phase and the oil phase are mixed and homogenized for 10min under 25Hz and are then cooled to 45°C, the chelator, the hydrolyzed soy protein complex, the arginine and the rose oil are added and are stirred under 30Hz, and after it is detected by sampling as qualified, filtration is carried out by a 200-mesh filter cloth to obtain the face cream.

### Example 4

Example 4 of the invention provides a skin-care composition for tightening skin. The skin-care composition for tightening skin comprises, by weight, 6.7 parts of an emulsifier, 3 parts of an acmella oleracea extract, 0.2 parts of hydrogenated lecithin (CAS No.92128-87-5), 1 part of trehalose (CAS No.99-20-7), 2.5 parts of a hydrolyzed soy protein complex, 1 part of a complex plant extract, 1 part of a caffeine liposome, and 2 parts of a trifluoroacetyl tripeptide-2 complex.

The emulsifier consists of, by weight, 2 parts of glyceryl stearate/PEG-100 stearate, 4.5 parts of cetostearyl alcohol (CAS No.67762-27-0), and 0.2 parts of sodium stearoyl glutamate (CAS No.38517-23-6).

The glyceryl stearate/PEG-100 stearate is purchased from Shanghai Goodway industrial Co., Ltd. under the designation of ARLACEL 170.

The acmella oleracea extract is purchased from PUEN Biotech (Shanghai) Co., Ltd., under the designation of Gatuline IN-Tense.

The hydrolyzed soy protein complex is purchased from Shanghai YaoLan Trading Co., Ltd. under the designation of Firming Toner.

The complex plant extract comprises an aloe vera leaf extract and a brassica oleracea italica extract and is purchased from Shanghai RuiMu Chemical Co,. Ltd. under the designation of BioDtox.

The caffeine liposome is purchased from Shanghai HaoYun Chemical Co., Ltd. under the designation of LIPO COFFIN HERBASOME

The trifluoroacetyl tripeptide-2 complex is purchased from Shanghai ZhiRou Chemical Co., Ltd. under the designation of Progline.

This example provides a face cream including the skin-care composition for tightening skin. The face cream is prepared from, by weight, 13.4% of the skin-care composition, 0.4% of a thickener, 16% of a moisturizer, 0.2% of an antioxidant, 11.1% of an emollient, 1% of a chelator, 0.48% of a skin conditioner, and the balance deionized water.

The thickener is Carbomer (CAS No.9007-20-9).

The moisturizer consists of, by weight, 8 parts of methylpropanediol (CAS No.2163-42-0) and 8 parts of 1,3-propanediol (CAS No.504-63-2).

The antioxidant is p-hydroxyacetophenone (CAS No.99-93-4).

The emollient consists of, by weight, 2 parts of butyrospermum parkii resin, 1 part of simmondsia chinensis seed oil, 1 part of squalane (CAS No.111-01-3), 5 parts of polydimethylsiloxane (CAS No. 9006-65-9), 2 parts of caprylyl methicone (CAS No. 17955-88-3), and 0.1 parts of tocopheryl acetate.

The butyrospermum parkii resin is purchased from Sanrise (Shanghai) Chemical Co., Ltd.

The simmondsia chinensis seed oil is purchased from FloraSun (Shanghai) Corporation.

The tocopheryl acetate is purchased from Shanghai ShangDe Chemical Co., Ltd.

The chelator is purchased from Shanghai HaoYun Chemical Co., Ltd. under the designation of PHL.

The skin conditioner consists of, by weight, 0.2 parts of allantoin (CAS. No.97-59-6), 0.2 parts of arginine (CAS No.74-79-3), and 0.08 parts of rose oil.

The rose oil is purchased from Ungerer And Company (Shanghai) Co., Ltd.

This example further provides a method for preparing the face cream. The method comprises the following steps:

Pre-treatment of an aqueous-phase: the thickener is added into the deionized water to be homogenized under 20Hz, then the moisturizer, the allantoin, the trehalose, the hydrogenated lecithin and the antioxidant are added, stirred under 30Hz and are heated to 83°C, and then the sodium stearoyl glutamate is added;

Pre-treatment of an oil phase: all components, except the sodium stearoyl glutamate, of the emulsifier and the emollient are stirred under 25Hz and are heated to 83°C, and then the acmella oleracea extract is added; and

After-treatment: the aqueous phase and the oil phase are mixed and homogenized for 10min under 25Hz and are then cooled to 45°C, the chelator, the hydrolyzed soy protein complex, the arginine, the complex plant extract, the caffeine liposome, the trifluoroacetyl tripeptide-2 complex and the rose oil are added and are stirred under 30Hz, and after it is detected by sampling as qualified, filtration is carried out by a 200-mesh filter cloth to obtain the face cream.

### Example 5

Example 5 of the invention provides a skin-care composition for tightening skin. The skin-care composition for tightening skin comprises, by weight, 4.16 parts of an emulsifier, 2 parts of an acmella oleracea extract, 0.1 parts of hydrogenated lecithin (CAS No.92128-87-5), 0.7 parts of trehalose (CAS No.99-20-7), 2 parts of a hydrolyzed soy protein complex, 0.5 parts of a complex plant extract, 0.7 parts of caffeine liposome, and 1.2 parts of a trifluoroacetyl tripeptide-2 complex.

The emulsifier consists of, by weight, 1 parts of glyceryl stearate/PEG-100 stearate, 3 parts of cetostearyl alcohol (CAS No.67762-27-0), and 0.6 parts of sodium stearoyl glutamate (CAS No.38517-23-6).

The glyceryl stearate/PEG-100 stearate is purchased from Shanghai Goodway industrial Co., Ltd. under the designation of ARLACEL 170.

The acmella oleracea extract is purchased from PUEN Biotech (Shanghai) Co., Ltd., under the designation of Gatuline IN-Tense.

The hydrolyzed soy protein complex is purchased from Shanghai YaoLan Trading Co., Ltd. under the designation of Firming Toner.

The complex plant extract comprises an aloe vera leaf extract and a brassica oleracea italica extract and is purchased from Shanghai RuiMu Chemical Co,. Ltd. under the designation of BioDtox.

The caffeine liposome is purchased from Shanghai HaoYun Chemical Co., Ltd. under the designation of LIPO COFFIN HERBASOME

The trifluoroacetyl tripeptide-2 complex is purchased from Shanghai ZhiRou Chemical Co., Ltd. under the designation of Progline.

This example provides a face cream including the skin-care composition for tightening skin. The face cream is prepared from, by weight, 11.36% of the skin-care composition, 0.1% of a thickener, 15% of a moisturizer, 0.1% of an antioxidant, 8.1% of an emollient, 0.5% of a chelator, 0.2% of a skin conditioner, and the balance deionized water.

The thickener is Carbomer (CAS No.9007-20-9).

The moisturizer consists of, by weight, 7.5 parts of methylpropanediol (CAS No.2163-42-0) and 7.5 parts of 1,3-propanediol (CAS No.504-63-2).

The antioxidant is p-hydroxyacetophenone (CAS No.99-93-4).

The emollient consists of, by weight, 1 part of butyrospermum parkii resin, 1 part of simmondsia chinensis seed oil, 1 part of squalane (CAS No.111-01-3), 4 parts of polydimethylsiloxane (CAS No. 9006-65-9), 1 part of caprylyl methicone (CAS No. 17955-88-3), and 0.1 parts of tocopheryl acetate.

The butyrospermum parkii resin is purchased from Sanrise (Shanghai) Chemical Co., Ltd.

The simmondsia chinensis seed oil is purchased from FloraSun (Shanghai) Corporation.

The tocopheryl acetate is purchased from Shanghai ShangDe Chemical Co., Ltd.

The chelator is purchased from Shanghai HaoYun Chemical Co., Ltd. under the designation of PHL.

The skin conditioner consists of, by weight, 0.05 parts of allantoin (CAS. No.97-59-6), 0.05 parts of arginine (CAS No.74-79-3), and 0.08 parts of rose oil.

The rose oil is purchased from Ungerer And Company (Shanghai) Co., Ltd.

This example further provides a method for preparing the face cream. The method comprises the following steps:

Pre-treatment of an aqueous-phase: the thickener is added into the deionized water to be homogenized under 15Hz, then the moisturizer, the allantoin, the trehalose, the hydrogenated lecithin and the antioxidant are added, stirred under 25Hz and are heated to 80°C, and then the sodium stearoyl glutamate is added;

Pre-treatment of an oil phase: all components, except the sodium stearoyl glutamate, of the emulsifier and the emollient are stirred under 20Hz and are heated to 80°C, and then the acmella oleracea extract is added; and

After-treatment: the aqueous phase and the oil phase are mixed and homogenized for 10min under 20Hz and are then cooled to 40°C, the chelator, the hydrolyzed soy protein complex, the arginine, the complex plant extract, the caffeine liposome, the trifluoroacetyl tripeptide-2 complex and the rose oil are added and are stirred under 25Hz, and after it is detected by sampling as qualified, filtration is carried out by a 200-mesh filter cloth to obtain the face cream.

### Example 6

Example 6 of the invention provides a skin-care composition for tightening skin. The skin-care composition for tightening skin comprises, by weight, 8.2 parts of an emulsifier, 4 parts of an acmella oleracea extract, 0.45 parts of hydrogenated lecithin (CAS No.92128-87-5), 1.5 parts of trehalose (CAS No.99-20-7), 3 parts of a hydrolyzed soy protein complex, 2 parts of a complex plant extract, 1 part of a caffeine liposome, and 3 parts of a trifluoroacetyl tripeptide-2 complex.

The emulsifier consists of, by weight, 3 parts of glyceryl stearate/PEG-100 stearate, 5 parts of cetostearyl alcohol (CAS No.67762-27-0), and 0.2 parts of sodium stearoyl glutamate (CAS No.38517-23-6).

The glyceryl stearate/PEG-100 stearate is purchased from Shanghai Goodway industrial Co., Ltd. under the designation of ARLACEL 170.

The acmella oleracea extract is purchased from PUEN Biotech (Shanghai) Co., Ltd., under the designation of Gatuline IN-Tense.

The hydrolyzed soy protein complex is purchased from Shanghai YaoLan Trading Co., Ltd. under the designation of Firming Toner.

The complex plant extract comprises an aloe vera leaf extract and a brassica oleracea italica extract and is purchased from Shanghai RuiMu Chemical Co., Ltd. under the designation of BioDtox.

The caffeine liposome is purchased from Shanghai HaoYun Chemical Co., Ltd., under the designation of LIPO COFFIN HERBASOME

The trifluoroacetyl tripeptide-2 complex is purchased from Shanghai ZhiRou Chemical Co., Ltd., under the designation of Progline.

This example provides a face cream including the skin-care composition for tightening skin. The face cream is prepared from, by weight, 23.15% of the skin-care composition, 0.5% of a thickener, 20% of a moisturizer, 0.5% of an antioxidant, 19.3% of an emollient, 2% of a chelator, 0.8% of a skin conditioner, and the balance deionized water.

The thickener is Carbomer (CAS No.9007-20-9).

The moisturizer consists of, by weight, 10 parts of methylpropanediol (CAS No.2163-42-0) and 10 parts of 1,3-propanediol (CAS No.504-63-2).

The antioxidant is p-hydroxyacetophenone (CAS No.99-93-4).

The emollient consists of, by weight, 4 parts of butyrospermum parkii resin, 3 parts of simmondsia chinensis seed oil, 2 parts of squalane (CAS No.111-01-3), 6 parts of polydimethylsiloxane (CAS No. 9006-65-9), 4 parts of caprylyl methicone (CAS No. 17955-88-3), and 0.3 parts of tocopheryl acetate.

The butyrospermum parkii resin is purchased from Sanrise (Shanghai) Chemical Co., Ltd.

The simmondsia chinensis seed oil is purchased from FloraSun (Shanghai) Corporation.

The tocopheryl acetate is purchased from Shanghai ShangDe Chemical Co., Ltd.

The chelator is purchased from Shanghai HaoYun Chemical Co., Ltd. under the designation of PHL.

The skin conditioner consists of, by weight, 0.3 parts of allantoin (CAS. No.97-59-6), 0.35 parts of arginine (CAS No.74-79-3), and 0.15 parts of rose oil.

The rose oil is purchased from Ungerer And Company (Shanghai) Co., Ltd.

This example further provides a method for preparing the face cream. The method comprises the following steps:

Pre-treatment of an aqueous-phase: the thickener is added into the deionized water to be homogenized under 25Hz, then the moisturizer, the allantoin, the trehalose, the hydrogenated lecithin and the antioxidant are added, stirred under 35Hz and are heated to 85°C, and then the sodium stearoyl glutamate is added;

Pre-treatment of an oil phase: all components, except the sodium stearoyl glutamate, of the emulsifier and the emollient are stirred under 30Hz and are heated to 85°C, and then the acmella oleracea extract is added; and

After-treatment: the aqueous phase and the oil phase are mixed and homogenized for 15min under 30Hz and are then cooled to 50°C, the chelator, the hydrolyzed soy protein complex, the arginine, the complex plant extract, the caffeine liposome, the trifluoroacetyl tripeptide-2 complex and the rose oil are added and are stirred under 35Hz, and after it is detected by sampling as qualified, filtration is carried out by a 200-mesh filter cloth to obtain the face cream.

### Example 7

Example 7 of the invention provides a skin-care composition for tightening skin. The skin-care composition in this example is identical in specific implementation with the skin-care composition in Example 4 and differs from the skin-care composition in Example 4 in that the part by weight of the sodium stearoyl glutamate is 1.5.

This example provides a face cream comprising the skin-care composition for tightening skin. The face cream in this example is identical in specific implementation with the face cream in Example 4 and differs from the face cream in Example 4 in that the percentage by weight of the skin-care composition is 18.7%.

This example further provides a method for preparing the face cream. The method is identical in specific implementation with the method in Example 4.

### Example 8

Example 8 of the invention provides a skin-care composition for tightening skin. The skin-care composition in this example is identical in specific implementation with the skin-care composition in Example 4 and differs from the skin-care composition in Example 4 in that the glyceryl stearate/PEG-100 stearate is replaced with polyethylene glycol laurate (CAS No.31943-11-0).

This example provides a face cream comprising the skin-care composition for tightening skin. The face cream in this example is identical in specific implementation with the face cream in Example 4.

This example further provides a method for preparing the face cream. The method is identical in specific implementation with the method in Example 4.

### Example 9

Example 9 of the invention provides a skin-care composition for tightening skin. The skin-care composition in this example is identical in specific implementation with the skin-care composition in Example 4 and differs from the skin-care composition in Example 4 in that the sodium stearoyl glutamate is replaced with sodium lauryl sulfate (CAS No. 151-21-3).

This example provides a face cream comprising the skin-care composition for tightening skin. The face cream in this example is identical in specific implementation with the face cream in Example 4.

This example further provides a method for preparing the face cream. The method is identical in specific implementation with the method in Example 4.

### Example 10

Example 10 of the invention provides a skin-care composition for tightening skin. The skin-care composition in this example is identical in specific implementation with the skin-care composition in Example 4 and differs from the skin-care composition in Example 4 in that the skin-care composition does not include hydrogenated lecithin.

This example provides a face cream comprising the skin-care composition for tightening skin. The face cream in this example is identical in specific implementation with the face cream in Example 4 and differs from the face cream in Example 4 in that the percentage by weight of the skin-care composition is 17.2%.

This example further provides a method for preparing the face cream. The method is identical in specific implementation with the method in Example 4.

### Example 11

Example 11 of the invention provides a skin-care composition for tightening skin. The skin-care composition in this example is identical in specific implementation with the skin-care composition in Example 4 and differs from the skin-care composition in Example 4 in that the skin-care composition does not include trehalose.

This example provides a face cream comprising the skin-care composition for tightening skin. The face cream in this example is identical in specific implementation with the face cream in Example 4 and differs from the face cream in Example 4 in that the percentage by weight of the skin-care composition is 16.4%.

This example further provides a method for preparing the face cream. The method is identical in specific implementation with the method in Example 4.

### Example 12

Example 12 of the invention provides a skin-care composition for tightening skin. The skin-care composition in this example is identical in specific implementation with the skin-care composition in Example 4 and differs from the skin-care composition in Example 4 in that the part by weight of trehalose is 3.

This example provides a face cream comprising the skin-care composition for tightening skin. The face cream in this example is identical in specific implementation with the face cream in Example 4 and differs from the face cream in Example 4 in that the percentage by weight of the skin-care composition is 19.4%.

This example further provides a method for preparing the face cream. The method is identical in specific implementation with the method in Example 4.

### Example 13

Example 13 of the invention provides a skin-care composition for tightening skin. The skin-care composition in this example is identical in specific implementation with the skin-care composition in Example 4 and differs from the skin-care composition in Example 4 in that the part by weight of the hydrolyzed soy protein complex is 6.

This example provides a face cream comprising the skin-care composition for tightening skin. The face cream in this example is identical in specific implementation with the face cream in Example 4 and differs from the face cream in Example 4 in that the percentage by weight of the skin-care composition is 20.9%.

This example further provides a method for preparing the face cream. The method is identical in specific implementation with the method in Example 4.

### Example 14

Example 14 of the invention provides a skin-care composition for tightening skin. The skin-care composition in this example is identical in specific implementation with the skin-care composition in Example 4 and differs from the skin-care composition in Example 4 in that the part by weight of complex plant extract is 0.1.

This example provides a face cream comprising the skin-care composition for tightening skin. The face cream in this example is identical in specific implementation with the face cream in Example 4 and differs from the face cream in Example 4 in that the percentage by weight of the skin-care composition is 16.5%.

This example further provides a method for preparing the face cream. The method is identical in specific implementation with the method in Example 4.

### Example 15

Example 15 of the invention provides a skin-care composition for tightening skin. The skin-care composition in this example is identical in specific implementation with the skin-care composition in Example 4 and differs from the skin-care composition in Example 4 in that the skin-care composition does not include the complex plant extract or the caffeine liposome.

This example provides a face cream comprising the skin-care composition for tightening skin. The face cream in this example is identical in specific implementation with the face cream in Example 4 and differs from the face cream in Example 4 in that the percentage by weight of the skin-care composition is 15.4%.

This example further provides a method for preparing the face cream. The method is identical in specific implementation with the method in Example 4.

### Example 16

Example 16 of the invention provides a skin-care composition for tightening skin. The skin-care composition in this example is identical in specific implementation with the skin-care composition in Example 4 and differs from the skin-care composition in Example 4 in that the part by weight of the trifluoroacetyl tripeptide-2 complex is 5.

This example provides a face cream comprising the skin-care composition for tightening skin. The face cream in this example is identical in specific implementation with the face cream in Example 4 and differs from that in Example 4 in that the percentage by weight of the skin-care composition is 20.4%.

This example further provides a method for preparing the face cream. The method is identical in specific implementation with the method in Example 4.

### Example 17

This example provides a face cream comprising the skin-care composition for tightening skin. The face cream in this example is identical in specific implementation with the face cream in Example 4 and differs from the face cream in Example 4 in that the face cream does not include the skin-care composition.

This example further provides a method for preparing the face cream. The method is identical in specific implementation with the method in Example 4.

### Performance evaluation

1. Stability test: the face creams provided by Examples 1-17 are subjected to stability test as samples.
   (1) Heat resistance test: the samples are placed in an electric-heating constant-temperature incubator at a temperature of (40±1)°C for 24h, and whether or not the samples become thinner or layered, or are changed in color and hardness after recovering to the room temperature is observed to estimate the heat resistance of the samples;
   (2) Cold resistance test: the samples are placed in an electric refrigerator at a temperature of (-8±2)°C for 24h, and whether or not the samples become thinner or layered, or are changed in color and hardness after recovering to the room temperature is observed to estimate the cold resistance of the samples;
   (3) Centrifugal test: the samples are placed in a centrifugal machine and are tested for 30min at a speed of (2000-4000)r/min, and then separation and layering conditions of the samples are observed to estimate the mechanical stability of the samples.

The samples are graded into levels 0-4 in total according to thinning, color changing, layering and hardness changing conditions of the samples before and after tests, wherein level 0 refers to no thinning, color changing, layering or hardness changing, level 1 refers to slight thinning, color changing, layering or hardness changing, level 2 refers to obvious color changing and thinning, no layering and slight hardness changing, level 3 refers to slight layering and slight hardness changing, and level 4 refers to obvious layering and hardness changing. The heat stability, cold stability and mechanical stability of the compositions are shown in Table 1.

2. Sensitivity test: the face creams provided by Examples 1-17 are subjected to a sensitivity test particularly as follows: people with oily skin and sensitive skin are selected to receive a patch test, the testees should have no scars, or too many bruises or hair on the skin, and people who have a history of severe allergies or are seriously allergic to cosmetics should not participate in the test; the sensitivity of the face creams provided by Examples 1-16 is evaluated according to skin conditions of tested parts of the testees with oily skin and sensitive skin, wherein the evaluation indexes include itching, tightness, irritation, reddening, dry sensation, and inflammation; and levels 0-4 are set in total, wherein level 0: no sensitization; level 1: dry skin and slight itching; level 2: slight skin reddening and severe itching; level 3: slight erythema and redness; level 4: severe erythema and redness, and inflammation. Test results are shown in Table 1.

### 3. Pore firming effect and relapse test

Test method: the face creams provided by Examples 1-17 are used once by 33 Chinese health females, aging from 25-55, with normal skin (pores on faces are coarse) and without distinct pore differences between the left half and the right half of the face, and the pores of the testees are detected through an instrument (VISIA-CR). Comparison is carried out according to image analysis results and evaluation results obtained before and after usage through a statistical inspection method to determine whether or not there is a statistical difference.

Statistical method: if data distribution accords with normal distribution, test results are inspected by pairing t, and the inspection level meets α = 0.05; if the data distribution does not accord with normal distribution, the test results are inspected by non-parameter inspection Wilicoxon, and the inspection level meets α = 0.05.

Test environment: the temperature of the test environment is 20.0°C - 21.8°C, and the relative humidity of the test environment is 48.0% - 54.0%.

Test indexes: the front side, the left side by 45°, and the right side by 45° of the face.

Test procedure: the testees washed faces with a face cleansing product, then dried the faces with dry facial tissues, and sit in a laboratory under 21±1°C and 50±5% RH for 30min; face images were shot (VISIA-CR); the testees used the face creams provided by Examples 1-17, and face images were shot again 15 min later (VISIA-CR).

### Pore firming effect test

Face images of the testees were shot before the testees used ImL of the face creams provided by Examples 1 - 17, face images were shot again 15min after the testees used the face creams, and the pore improvement conditions were evaluated by self-evaluation. Test results are shown in Table 2.
(1) Effect standards:
   a. The face images of the testees before and after usage of the face creams were analyzed, the ratio of the pore area to an analysis area of each testee before and after usage of the face creams provided by Examples 1-17 was calculated, and the pore reduction rate was analyzed, wherein the pore reduction rate=(the ratio of the pore area to the analysis area before usage of the face creams provided by Examples 1-17 - the ratio of the pore area to the analysis area after usage of the face creams provided by Examples 1-17)/the ratio of the pore area to the analysis area before usage of the face creams provided by Examples 1-17.
   b. Self-evaluation standards for the testees: score 1: no improvement on the pore firming effect after usage; score 2: non-obvious improvement on the pore firming effect after usage; score 3: moderate improvement on the pore firming effect after usage; score 4: obvious improvement on the pore firming effect after usage; score 5: great improvement on the pore firming effect after usage. Then, the degree of satisfaction with the products is summarized and is represented by the percentage of testees giving a self-evaluation score of 5 in all the testees.
(2) Relapse test

Face images of the testees after the testees use 1ml of face creams provided by Examples 4-6 for 15 days were shot, face images of the testees after the testees stop using the face creams for 15 days were also shot, and it is found that the pore area barely changed before and after the testees stop using the face creams.

4. Sensory test: the face creams provided by Examples 1-17 were subjected to a sensory test particularly as follows: before using the samples, the testees cleaned their faces with the same mild face cleansing product, then applied a toner and a lotion to the faces and smeared the face creams provided by Examples 1-17 on the faces once every day in the morning for two weeks, and then sensory evaluation was carried out; the evaluation indexes mainly include the anti-wrinkle effect and the anti-aging effect; the face creams are graded into levels 1-5 in total, wherein level 1 refers to no improvement, level 2 refers to non-obvious improvement, level 3 refers to moderate improvement, level 4 refers to good improvement, level 5 refers to remarkable improvement, and test results are shown in Table 2.

5. Physical and chemical property test: physical and chemical properties of the face cream provided by Example 4 were tested:
(1) Appearance: the face cream provided by Example 4 was faint yellow fine paste;
(2) Fragrance: the fragrance of the face cream provided by Example 4 was evaluated by sensation and accords with a specified fragrance (rose);
(3) pH test: the face cream provided by Example 4 was tested by a pH meter through a pH test method according to GB T13531.1-2008, and the result showed that the pH of the face cream is 6.02;
(4) Relative density: the relative density of the face cream provided by Example 4 was tested by a density gauge under 20°C through a relative density test method according to GB T13531.4-2013, and the test result showed that the relative density is 1.00g/cm³.

**Table 1: Performance characteristic test**

| | Stability test | | | Sensitivity test | |
|---|---|---|---|---|---|
| | Heat stability | Cold stability | Mechanical stability | Sensitive skin | Oily skin |
| Example 1 | Level 2 | Level 2 | Level 2 | Level 3 | Level 2 |
| Example 2 | Level 1 | Level 1 | Level 1 | Level 3 | Level 2 |
| Example 3 | Level 1 | Level 0 | Level 1 | Level 1 | Level 0 |
| Example 4 | Level 0 | Level 0 | Level 0 | Level 0 | Level 0 |
| Example 5 | Level 1 | Level 0 | Level 0 | Level 0 | Level 0 |
| Example 6 | Level 0 | Level 0 | Level 0 | Level 0 | Level 0 |
| Example 7 | Level 3 | Level 2 | Level 2 | Level 2 | Level 1 |
| Example 8 | Level 2 | Level 1 | Level 1 | Level 1 | Level 0 |
| Example 9 | Level 2 | Level 1 | Level 2 | Level 2 | Level 1 |
| Example 10 | Level 1 | Level 0 | Level 1 | Level 3 | Level 2 |
| Example 11 | Level 1 | Level 1 | Level 1 | Level 3 | Level 2 |
| Example 12 | Level 1 | Level 0 | Level 0 | Level 2 | Level 1 |
| Example 13 | Level 2 | Level 1 | Level 1 | Level 2 | Level 1 |
| Example 14 | Level 1 | Level 0 | Level 0 | Level 0 | Level 0 |
| Example 15 | Level 1 | Level 0 | Level 0 | Level 1 | Level 1 |
| Example 16 | Level 1 | Level 0 | Level 1 | Level 1 | Level 1 |
| Example 17 | Level 4 | Level 4 | Level 4 | Level 4 | Level 4 |

**Table 2: Performance characteristic test**

| | Pore firming effect test | | Sensory evaluation | |
|---|---|---|---|---|
| | Pore reduction rate | Degree of satisfaction | Anti-wrinkle | Anti-aging |
| Example 1 | 1.13% | 49% | Level 2 | Level 2 |
| Example 2 | 1.44% | 65% | Level 3 | Level 3 |
| Example 3 | 1.85% | 77% | Level 4 | Level 4 |
| Example 4 | 2.36% | 93% | Level 5 | Level 5 |
| Example 5 | 2.17% | 84% | Level 4 | Level 5 |
| Example 6 | 2.25% | 89% | Level 5 | Level 5 |
| Example 7 | 1.68% | 72% | Level 3 | Level 4 |
| Example 8 | 1.91% | 79% | Level 4 | Level 4 |
| Example 9 | 1.82% | 76% | Level 3 | Level 4 |
| Example 10 | 2.03% | 81% | Level 4 | Level 4 |
| Example 11 | 1.98% | 80% | Level 4 | Level 4 |
| Example 12 | 2.09% | 82% | Level 4 | Level 4 |
| Example 13 | 1.94% | 79% | Level 4 | Level 4 |
| Example 14 | 2.11% | 82% | Level 4 | Level 4 |
| Example 15 | 1.88% | 78% | Level 3 | Level 4 |
| Example 16 | 2.18% | 84% | Level 4 | Level 5 |
| Example 17 | 0.48% | 18% | Level 1 | Level 2 |

As can be seen from the test results in Table 1 and Table 2, the skin-care composition for tightening skin of the invention has high heat stability, cold stability and mechanical stability, and a skin-care product containing the skin-care composition will not irritate skin, is suitable for people with sensitive kin and oily skin, has good effects in tightening skin, preventing wrinkles and resisting aging, and can avoid relapses.

The aforesaid embodiments are only illustrative ones which are used for explaining some characteristics of the method of the invention. The appended claims are intended to cover a range as wide as possible, and the embodiments illustrated in the specification are selected from the combinations of all possible ones to explain the implementations. Therefore, the claims should not be interpreted as limitations of the illustrative characteristics of the invention, as intended by the applicant. Value ranges involved in the claims include sub-ranges thereof, and variations within these ranges are included in the claims where possible.

## Claims

1. A skin-care composition for tightening skin, being prepared from an emulsifier and an acmella oleracea extract, wherein the emulsifier comprises one or more selected from the group consisting of stearate, stearyl alcohol and stearyl glucoside.

2. The skin-care composition for tightening skin according to claim 1, **characterized in that** the emulsifier further comprises stearyl glutamate that accounts for 2-4wt% of the emulsifier.

3. The skin-care composition for tightening skin according to claim 2, **characterized in that** the stearyl glutamate accounts for 3-10wt% of the acmella oleracea extract.

4. The skin-care composition for tightening skin according to claim 2, **characterized in that** the skin-care composition is further prepared from hydrogenated lecithin, trehalose and a hydrolyzed soy protein complex.

5. The skin-care composition for tightening skin according to claim 4, **characterized in that** the weight ratio of the trehalose and the hydrolyzed soy protein complex is 1: (2-3).

6. The skin-care composition for tightening skin according to claim 2 or 4, **characterized in that** the skin-care product is further prepared from a complex plant extract, a caffeine liposome and a trifluoroacetyl tripeptide-2 complex.

7. The skin-care composition for tightening skin according to claim 6, **characterized in that** the weight ratio of the complex plant extract, the caffeine liposome and the trifluoroacetyl tripeptide-2 complex is 1 : (0.5-1.5) : (1.5-2.5).

8. A use of the skin-care composition for tightening skin according to any one of claims 1-7, wherein the skin-care composition is applied to a skin-care product, and the skin-care product is one or more selected from a lotion, an emulsion, a face cream, an eye cream, a face cleanser, a face mask, a BB cream, a pure essence, a sunscreen scream, a sunscreen lotion, an acne lotion, a pimple lotion, a cleansing toner, a cleansing lotion, an essential oil, a shampoo, a moisturizer, a toner, an astringent and a softening lotion.

9. The application of the skin-care composition for tightening skin according to claim 8, wherein the skin-care product is a face cream which is prepared from, by weight, 9%-25% of the skin-care composition, 0.1%-0.5% of a thickener, 15%-20% of a moisturizer, 0-0.5% of an antioxidant, 8%-20% of an emollient, 0.5%-2% of a chelator, 0.2%-0.8% of a skin conditioner, and the balance deionized water.

10. A method for preparing a face cream, wherein the face cream is prepared from, by weight, 9%-25% of a skin-care composition, 0.1%-0.5% of a thickener, 15%-20% of a moisturizer, 0-0.5% of an antioxidant, 8%-20% of an emollient, 0.5%-2% of a chelator, 0.2%-0.8% of a skin conditioner, and the balance deionized water;
the method for preparing the face cream comprises the following steps:
pre-treatment of an aqueous phase: after the thickener and the deionized water are homogenized, the moisturizer, the antioxidant and half of the skin conditioner by weight are added and are then stirred and heated;
pre-treatment of an oil phase: all components, except stearyl glutamate, of the emulsifier, and the emollient are stirred and heated, and then an acmella oleracea extract is added; and
after-treatment: the aqueous phase and the oil phase are mixed, homogenized and cooled, the chelator, the rest of the skin conditioner and other compositions, except for trehalose, hydrogenated lecithin and an emulsifier, of the skin-care composition are added, stirred and filtered to obtain the face cream.
